# EUROPÄISCHE PATENTSCHRIFT

(11) **EP 0 603 695 B1**
(45) Veröffentlichungstag und Bekanntmachung des Hinweises auf die Patenterteilung: **02.05.1997**
(21) Anmeldenummer: 93120037.2
(22) Anmeldetag: 11.12.1993
(51) Int. Cl.: C07D 311/72

(54) **Verfahren zur Herstellung von Alpha-Tocopherol und Alpha-Tocopherylacetat in flüssigem oder überkritischem Kohlendioxid**
Process for the preparation of alpha-tocopherol and alpha-tocopheryl-acetate with liquid or supercritical carbondioxyde
Procédé de préparation de alpha-tocophérol et de alpha-tocophéryl acétate à l'aide de dioxyde de carbone liquide ou super-critique

(30) Priorität: 22.12.1992 DE 4243464
(43) Veröffentlichungstag der Anmeldung: 29.06.1994
(73) Patentinhaber: BASF Aktiengesellschaft, 67063 Ludwigshafen (DE)
(72) Erfinder: Lowack, Rainer, Dr., D-68167 Mannheim (DE); Meyer, Joachim, Dr., D-67133 Maxdorf (DE); Eggersdorfer, Manfred, Dr., D-67227 Frankenthal (DE); Grafen, Paul, Dr., D-67273 Weisenheim (DE)

(56) Entgegenhaltungen:
- CH-A- 222 170
- FR-A- 2 602 772

## Beschreibung

Die Erfindung betrifft ein Verfahren zur Herstellung von α-Tocopherol oder α-Tocopherylacetat durch Cyclokondensation von Trimethylhydrochinon mit Phytol oder Isophytol in Gegenwart eines sauren Katalysators und eines inerten Lösungsmittels und - falls gewünscht - anschließende Acetylierung.

Wegen des ständig steigenden Bedarfs an α-Tocopherol (Vitamin E) ist seine Herstellung durch die oben beschriebene Cyclokondensation von vielen Vitaminherstellern untersucht worden. So werden in der Patentliteratur zahlreiche Cyclokondensationsverfahren beschrieben, die sich im wesentlichen in der Art des sauren Katalysators oder in der Wahl der Lösungsmittel unterscheiden. Als saure Katalysatoren werden z.B. Gemische aus starken Säuren und Lewissäuren, wie HCl und ZnCl₂ (vgl. EP 100 471; JP-A2-226 976/87; JP-A2-54380/85), saure Ionenaustauscher (vgl. JP-A-77 064/78), mit Protonensäuren vorbehandeltes SiO₂/Al₂O₃ (vgl. DE 27 43 920 und DE 24 04 621), Trifluoressigsäure oder deren Anhydrid (vgl. EP 12824), bestimmte AlCl₃- oder BF₃-Komplexe (vgl. DE 19 09 164) oder BF₃ in Essigsäure (vgl. US 3 444 213) beschrieben.

Als geeignete Lösungsmittel sind beispielsweise Kohlenwasserstoffe, wie Heptan, halogenierte Kohlenwasserstoffe, Ketone, Essigsäure und insbesondere Essigsäurealkylester verwendet worden.

Das bei der Cyclokondensation von Trimethylhydrochinon und Isophytol entstehende Reaktionswasser wird in diesen Verfahren entweder azeotrop abdestilliert oder durch hygroskopische Substanzen, wie ZnCl₂ und Salzsäuregas in Form einer sich abscheidenden wäßrigen Phase gebunden.

Nachteilig an den bekannten Verfahren ist, daß die Aufarbeitung des Reaktionsgemisches sowie die Reinigung des Tocopherols bzw. des Tocopherylacetats in technischem Maßstab recht aufwendig sind. So muß das erhaltene rohe α-Tocopherol bzw. das α-Tocopherylacetat in den meisten Verfahren letztlich durch Destillation unter sehr stark vermindertem Druck gereinigt werden, was hinsichtlich der benötigten Energie und Apparaturen sehr aufwendig ist und wegen der benötigten Temperaturen von mehr als 200°C zu Ausbeuteverlusten führt.

Gemäß neueren Untersuchungen von G. Brunner et al in " The Journal of Supercritical Fluids", 1991, 4, Seiten 72-80, der Diplomarbeit von Martin Unterhuber, Universität Erlangen-Nürnberg, April 1986 und dem französischen Patent FR 2 602 772 lassen sich sowohl α-Tocopherol als auch Tocopherylacetat durch eine Destraktion in überkritischem Kohlendioxid reinigen. Das Prinzip einer Destraktion wird von K. Zosel in der Zeitschrift "Angewandte Chemie", 1978, 90, Seiten 748-55 (Intern. Edit. in Engl., 1978, Vol. 17, Seiten 702-709) beschrieben.

Wollte man die Reinigungsmethode für synthetisch hergestelltes α-Tocopherol verwenden, so müßte bei Anwendung der bisher bekannten Syntheseverfahren für Tocopherol bzw. Tocopherylacetat vor einer derartigen Reinigung das für die Synthesereaktion verwendete organische Lösungsmittel erst abgetrennt werden.

Es war die Aufgabe der Erfindung, die Herstellung von α-Tocopherol bzw. α-Tocopherylacetat durch Cyclokondensation von Trimethylhydrochinon und Phytol oder Isophytol in Gegenwart eines sauren Katalysators und ggf. anschließende Acetylierung so zu verbessern, daß die Aufarbeitung des Reaktionsgemisches und die Reinigung des Reaktionsproduktes in technischem Maßstab vorteilhafter gestaltet werden kann.

Gegenstand der Erfindung ist nun ein Verfahren zur Herstellung von α-Tocopherol oder α-Tocopherylacetat durch Cyclokondensation von Trimethylhydrochinon mit Phytol oder Isophytol in Gegenwart eines sauren Katalysators und eines Lösungsmittels und ggf. anschließende Acetylierung, das dadurch gekennzeichnet ist, daß man die Cyclokondensation und ggf. die anschließende Acetylierung in flüssigem oder - vorzugsweise - in überkritischem Kohlendioxid als Solvens durchführt.

Überkritisches Kohlendioxid ist wegen seiner physiologischen Unbedenklichkeit, seiner guten Lösefähigkeit für Phytol bzw. Isophytol und die Reaktionsprodukte sowie wegen seiner niedrigen kritischen Temperatur von nur 31°C und seinem niedrigen kritischen Druck ein gutes Lösungsmittel für die Cyclokondensation von Trimethylhydrochinon und Isophytol zu α-Tocopherol und ggf. dessen anschließende Acetylierung.

Besonders vorteilhaft gestaltet sich das Verfahren, wenn man dem flüssigen oder vorzugsweise dem überkritischen Kohlendioxid noch Ethan, Propan oder Butan, vorzugsweise Propan, in Mengen von etwa 10 bis 50 Gew.-%, vorzugsweise 15-30 Gew.-% als Co-Solvens zusetzt. Das Alkan dient während einer anschließenden Destraktion als Schleppmittel.

Durch die Zugabe von Ethan (kritische Temperatur 32°C), Propan (kritische Temperatur 97°C) oder Butan (kritische Temperatur 136°C) wird die Lösefähigkeit des überkritischen CO₂ noch wesentlich erhöht sowie die Viskosität des Reaktionsgemisches stark erniedrigt. Die erfindungsgemäß bevorzugt verwendeten Gemische von CO₂ und Propan sind bei Drücken oberhalb 65 bar und Temperaturen von oberhalb von 31°C im überkritischen Zustand. Die Gemischeigenschaften sind mit denen reiner überkritischer Stoffe vergleichbar.

Auch sehr vorteilhaft gestaltet sich das erfindungsgemäße Verfahren, wenn man die Cyclokondensation und ggf. die anschließende Acetylierung in überkritischem Kohlendioxid, dem bis zu 10 Gew.-% an Essigsäure zugesetzt wurde, durchführt.

Zur Durchführung der erfindungsgemäßen Synthese des Tocopherols werden Trimethylhydrochinon und Phytol oder Isophytol in flüssigem oder überkritischem CO₂ und oder einem CO₂/Co-Solvens-Gemisch zur Cyclokondensation gebracht. Da CO₂ eine kritische Temperatur von nur 31°C aufweist, wird die Cyclokondensation bevorzugt in überkritischem CO₂ durchgeführt. Bei Einsatz von flüssigem CO₂, also bei Temperaturen unterhalb von 31°C, müßte mit besonders aktiven Katalysatoren, wie Bortrifluorid oder Aluminiumtrichlorid, gearbeitet werden.

Als saure Katalysatoren für die Cyclokondensation können im wesentlichen alle für diese Umsetzung bekannten sauren Katalysatoren, d.h. sowohl Lewissäuren, wie Zinkchlorid, Alumosilikate, Zeolithe oder Montmorillonite als auch Bronstedsäuren, wie saure Ionenaustauscher, Toluolsulfonsäuren, Methansulfonsäure, Trifluormethansulfonsäure, Trifluoressigsäure, Schwefelsäure, Salzsäure, sulfonierte Aktivkohle oder auch Kombinationen dieser Säuren verwendet werden.

Mit besonderem Vorteil verwendet man als sauren Katalysator saure Ionenaustauscher oder ähnliche heterogene Katalysatoren, wie sulfonierte Aktivkohle oder Alumosilikate, da sich bei deren Verwendung keine wäßrige Phase ausbildet und man ohne die Notwendigkeit, das Reaktionswasser abzudestillieren, auskommt, wodurch sich das Verfahren wesentlich vereinfacht. Das bei der Reaktion gebildete Wasser kann nach der Cyclokondensation beispielsweise durch Zugabe von Acetanhydrid unter Regenerierung (Trocknung) des Katalysators entfernt werden.

Als saure Ionenaustauscher sind insbesondere stark saure Ionenaustauscher auf Polystyrolbasis enthaltend -SO₃H-Gruppen in der H⁺-Form geeignet. Genannt seien beispielsweise Amberlite® IR 112 und 120 (Rohm & Haas), Dowex® 50 (DOW Chemicals), Lewatit® S 100 (Bayer), Permutit® Q und RS (Permutit Co.), Wofatit® KPS 200 (Farbenfabrik Wolfen), Zeocarb® 225 (Permutit Co.) und insbesondere Amberlyst® A 15 (Rohm & Haas).

Aus dem gebildeten Tocopherol kann - falls gewünscht - ohne Austausch des Lösungsmittels und Katalysators durch anschließende Zugabe eines Acetylierungsmittels, wie Acetanhydrid, das α-Tocopherylacetat hergestellt werden.

Sowohl die Cyclokondensation als auch die Acetylierung werden im allgemeinen bei Temperaturen von 25 bis 220°C, vorzugsweise 90 bis 120°C und bei Drucken von 50 bis 300 bar, vorzugsweise 80 bis 200 bar durchgeführt.

Die Reaktionszeiten betragen im allgemeinen etwa 1 Minute bis 2 Stunden, vorzugsweise etwa 10 Minuten bis 1,5 Stunden.

Das Kohlendioxid verwendet man im allgemeinen in Mengen von 2 g bis 30 g, vorzugsweise 5 bis 20 g pro g Trimethylhydrochinon.

Bei Mitverwendung von Co-Solventien verringert sich das benötigte Kohlendioxid entsprechend.

Die erfindungsgemäß erhaltene Lösung von α-Tocopherol oder α-Tocopherylacetat in flüssigem oder überkritischem CO₂ oder in dem überkritischem Gemisch aus CO₂ und dem Co-Solvens kann anschließend sofort, d.h. ohne die Notwendigkeit zum Austausch des Lösungsmittels einer Reinigung durch Destraktion in diesem Lösungsmittel oder Lösungsmittelgemisch unterzogen werden. Eine solche Reinigung durch Destraktion aus überkritischem CO₂ ist für α-Tocopherylacetat in FR 2 602 772 beschrieben. Sie beruht im wesentlichen auf der unterschiedlichen Löslichkeit der niedrigsiedenden Nebenprodukte, von α-Tocopherol oder α-Tocopherylacetat und den höhersiedenden Nebenprodukten in dem überkritischen Gas und auf Isolieren der Nebenprodukte bzw. des Wertproduktes durch Entspannen der erhaltenen "Fraktionen" auf Normaldruck oder aber durch Temperaturerhöhung bei Konstanthalten des Druckes. Man arbeitet hierbei im allgemeinen bei Temperaturen von etwa 35 bis 90°C und bei Drucken von etwa 80 bis 250 bar. Bezüglich näherer Einzelheiten über die Destraktion von α-Tocopherol bzw. α-Tocopherylacetat verweisen wir auf FR 2 602 772 und die oben zitierte Diplomarbeit von M. Unterhuber.

Durch Kombination des erfindungsgemäßen Verfahrens mit der Reinigung des rohen α-Tocopherols oder α-Tocopherylacetates durch Destraktion aus überkritischem CO₂ oder einem überkritischen Gemisch aus CO₂ und insbesondere Propan und Butan können die Cyclokondensation von Trimethylhydrochinon mit Phytol oder Isophytol und ggf. die anschließende Acetylierung sowie die Aufarbeitung des Reaktionsgemisches und die Reinigung des Wertproduktes auf besonders vorteilhafte Weise durchgeführt werden.

### Beispiel 1

15.02 g (98,8 mmol) Trimethylhydrochinon und 10,59 g des stark sauren Ionenaustauschers Amberlyst® A15 wurden in einem 300 ml Autoklaven vorgelegt und 116 g CO₂ einkondensiert. Nach Erwärmen auf 100°C wurde ein Druck von 185 bar erreicht. 37,80 ml (29,48 g; 99,6 mmol) Isophytol wurden innerhalb von 1,5 Stunden (h)bei 108°C mittels einer HPLC-Pumpe eingepreßt. Nach weiteren 30 Minuten (Min) Rühren bei 108°C wurde der Autoklav auf Raumtemperatur (RT) abgekühlt und entspannt. Der Ionenaustauscher wurde durch Zentrifugieren des Reaktionsaustrages vom Reaktionsprodukt abgetrennt. Die 48.9 g des isolierten hellgelben Öls enthielten 83 % Tocopherol, was einer Ausbeute von 84 % an reinem α-Tocopherol entspricht.

### Beispiel 2

14.92 g (98.2 mmol) Trimethylhydrochinon und 4.51 g des stark sauren Ionenaustauschers Amberlyst® A15 wurden in einem 300 ml Autoklaven vorgelegt und 105 g CO₂ einkondensiert. Nach Erwärmen auf 90°C wurde ein Druck von 165 bar erreicht. 37,90 ml (99.9 mmol) Isophytol wurden innerhalb von 1,5 h bei 90°C mittels einer HPLC-Pumpe eingepreßt. Nach weiteren 30 Min Rühren bei 90°C wurden 26.1 ml Acetanhydrid innerhalb von 5 Min zugepumpt. Nach 4 h wurde der Autoklav auf RT abgekühlt und entspannt. Der Ionenaustauscher wurde durch Zentrifugieren des Reaktionsaustrages oder nach Lösen des Austrages in Heptan durch Filtration vom Reaktionsprodukt abgetrennt. Ausbeute nach Destillation 31,0 g (68 %) eines hellgelben Öls mit einem Tocopherylacetat-Gehalt größer als 98 %.

### Beispiel 3

15,12 g (99,38 mmol) Trimethylhydrochinon und 4.89 g Zinkchlorid wurden in einem 300 ml Autoklaven vorgelegt und 3 bar Salzsäuregas aufgepreßt, sowie 118 g CO₂ einkondensiert. Nach Erwärmen auf 110°C wurde ein Druck von 175 bar erreicht. 37,80 ml (99.9 mmol) Isophytol wurden innerhalb von 1,5 h bei 90°C mittels einer HPLC-Pumpe eingepreßt. Nach weiteren 30 Min Rühren bei 110°C wurde der Autoklav auf RT abgekühlt und entspannt. Der Reaktionsaustrag wurde durch Aufnehmen in Heptan und Waschen mit wäßrigem Methanol und dann Wasser vom Katalysator abgetrennt. Ausbeute bezogen auf reines Tocopherol betrug 23,12 g, entsprechend 55 % der Theorie.

## Patentansprüche

1. Verfahren zur Herstellung von α-Tocopherol oder Tocopherylacetat durch Cyclokondensation von Trimethylhydrochinon mit Phytol oder Isophytol in Gegenwart eines sauren Katalysators und eines Lösungsmittels und ggf. anschließende Acetylierung, dadurch gekennzeichnet, daß man die Cyclokondensation und ggf. die anschließende Acetylierung in flüssigem oder überkritischem Kohlendioxid als Solvens durchführt.

2. Verfahren gemäß Anspruch 1, dadurch gekennzeichnet, daß man die Cyclokondensation und ggf. die anschließende Acetylierung in überkritischem Kohlendioxid durchführt.

3. Verfahren gemäß Anspruch 1, dadurch gekennzeichnet, daß man die Cyclokondensation und ggf. die anschließende Acetylierung in flüssigem oder überkritischem Kohlendioxid, dem 10 bis 50 Gew.-% an Ethan, Propan oder Butan als Co-Solvens zugesetzt wurde, durchführt.

4. Verfahren gemäß Anspruch 2, dadurch gekennzeichnet, daß man die Cyclokondensation und ggf. die anschließende Acetylierung in überkritischem Kohlendioxid, dem 15 bis 30 Gew.-% an Propan oder Butan als Co-Solvens zugesetzt wurde, durchführt.

5. Verfahren gemäß Anspruch 1, dadurch gekennzeichnet, daß man die Cyclokondensation und ggf. die anschließende Acetylierung in flüssigem oder überkritischem Kohlendioxid, dem bis zu 10 Gew.-% Essigsäure als Co-Solvens zugesetzt wurde, durchführt.

## Claims

1. A process for preparing α-tocopherol or tocopheryl acetate by cyclocondensation of trimethylhydroquinone with phytol or isophytol in the presence of an acid catalyst and of a solvent and, where appropriate, subsequent acetylation, which comprises carrying out the cyclocondensation and, where appropriate, the subsequent acetylation in liquid or supercritical carbon dioxide as solvent.

2. A process as claimed in claim 1, wherein the cyclocondensation and, where appropriate, the subsequent acetylation are carried out in supercritical carbon dioxide.

3. A process as claimed in claim 1, wherein the cyclocondensation and, where appropriate, the subsequent acetylation are carried out in liquid or supercritical carbon dioxide to which from 10 to 50% by weight of ethane, propane or butane has been added as cosolvent.

4. A process as claimed in claim 2, wherein the cyclocondensation and, where appropriate, the subsequent acetylation are carried out in supercritical carbon dioxide to which from 15 to 30% by weight of propane or butane has been added as cosolvent.

5. A process as claimed in claim 1, wherein the cyclocondensation and, where appropriate, the subsequent acetylation are carried out in liquid or supercritical carbon dioxide to which up to 10% by weight of acetic acid has been added as cosolvent.

## Revendications

1. Procédé pour la préparation de l'α-tocophérol ou de l'acétate de tocophéryle par cyclodensation de la triméthylhydroquinone avec le phytol ou l'isophytol en présence d'un catalyseur acide et d'un solvant éventuellement suivie d'une acétylation, caractérisé par le fait que l'on procède à la cyclocondensation et le cas échéant à l'acétylation subséquente dans du dioxyde de carbone liquéfié ou à l'état surcritique qui sert de solvant.

2. Procédé selon la revendication 1, caractérisé par le fait que l'on procède à la cyclocondensation et le cas échéant à l'acétylation subséquente dans le dioxyde de carbone à l'état surcritique.

3. Procédé selon la revendication 1, caractérisé par le fait que l'on procède à la cyclocondensation et le cas échéant à l'acétylation subséquente dans le dioxyde de carbone liquéfié ou surcritique auquel on a ajouté 10 à 50 % en poids d'éthane, de propane ou de butane en tant que tiers-solvant.

4. Procédé selon la revendication 2, caractérisé par le fait que l'on procède à la cyclocondensation et le cas échéant à l'acétylation subséquente dans le dioxyde de carbone à l'état surcritique auquel on a ajouté 15 à 30 % en poids de propane ou de butane en tant que tiers-solvant.

5. Procédé selon la revendication 1, caractérisé par le fait que l'on procède à la cyclocondensation et le cas échéant à l'acétylation subséquente dans le dioxyde de carbone liquéfié ou à l'état surcritique auquel on a ajouté 10 % en poids d'acide acétique en tant que tiers-solvant.
